(19) Europäisches Patentamt
European Patent Office
Office européen des brevets

(11) Veröffentlichungsnummer: **0 005 452**
**B1**

(12) **EUROPÄISCHE PATENTSCHRIFT**

(21) Anmeldenummer: **79101122.4**

(22) Anmeldetag: **12.04.79**

(51) Int. Cl.³: **C 07 C 69/007**, C 07 C 69/18, C 07 C 69/30, C 07 C 69/74, C 07 C 69/76, C 07 C 67/04

(54) **Acyloxy-2-butene, ihre Herstellung und ihre Verwendung zur Herstellung von 4-Acetoxytiglinaldehyd**

(30) Priorität: **05.05.78 DE 2819592**

(43) Veröffentlichungstag der Anmeldung:
**28.11.79 Patentblatt 79/24**

(45) Bekanntmachung des Hinweises auf die Patenterteilung:
**01.10.80 Patentblatt 80/20**

(84) Benannte Vertragsstaaten:
**BE CH DE FR GB IT**

(56) Entgegenhaltungen:
**DE - A - 2 261 224**
**DE - B - 2 200 124**
**US - A - 3 394 170**
**US - A - 4 006 136**

**Chemical Abstracts, Band 66, Nr. 23, 1967 COLUMBUS, OHIO, USA, Seite 9758, Spalte 1, Abstract Nr. 104 490 p — in Verbindung mit Substance-Index band 66, 1967, Seite 612 S,**

**Chemical Abstracts, Band 77, Nr. 19, 1972 COLUMBUS, OHIO, USA Seite 430, Abstract Nr. 129 943 t — in Verbindung mit Substance Index Band 77, 1972, Seite 899 CS.**

(73) Patentinhaber: **BASF Aktiengesellschaft**
**Carl-Bosch-Strasse 38**
**D - 6700 Ludwigshafen (DE)**

(72) Erfinder: **Weitz, Hans-Martin, Dr.**
**Auf dem Koeppel 40**
**D - 6702 Bad Duerkheim 1 (DE)**
**Fischer, Rolf, Dr.**
**Bergstrasse 98**
**D - 6900 Heidelberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 005 452 B1

Acyloxy-2-butene ihre Herstellung und ihre Verwendung zur Herstellung von 4-Acetoxytiglinaldehyd

Diese Erfindung betrifft neue Acyloxy-2-butene und ihre Herstellung durch Umsetzung von 1-Acyloxy-1.3-butadienen mit Carbonsäuren und Sauerstoff in Gegenwart von Katalysatoren.

1.4-Diacetoxy-2-buten, ein wichtiges Zwischenprodukt für die Herstellung von Butandiol, wird z.B. nach dem in der DT—AS 2 217 452 beschriebenen Verfahren durch Umsetzung von Butadien mit Essigsäure und Sauerstoff in Gegenwart von Palladium enthaltenden Katalysatoren erhalten. Nach diesem Verfahren lassen sich auch Isopren, 2.3-Dimethylbutadien und Piperylen in die entsprechenden 1.4-Diacetoxy-2-butene überführen.

Gegenstand dieser Erfindung sind die Acyloxy-2-butene der Formel

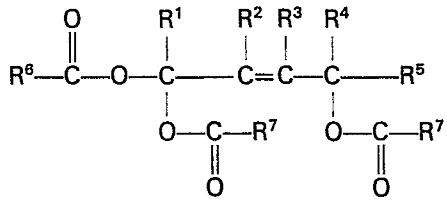

I,

in der $R^1$, $R^3$ und $R^4$ jeweils ein Wasserstoffatom oder einen Alkylrest mit 1 bis 5 C-Atomen, $R^2$ einen Alkylrest mit 1 bis 5 C-Atomen, $R^5$ ein Wasserstoffatom, einen Alkylrest mit 1 bis 5 C-Atomen oder eine $R^6$—CO—O-Gruppe und $R^6$ und $R^7$ jeweils ein Wasserstoffatom, einen Alkylrest mit 1 bis 5 C-Atomen oder einen Phenyl- oder Cyclohexylrest bedeuten. Diese neuen Acyloxy-2-butene sind wertvolle Zwischenprodukte, z.B. für die Herstellung von Terpenen und Pharmazeutika.

Die erfindungsgemäßen 1.1.4-Triacyloxy-2-alkyl-2-butene oder 1.1.4.4-Tetraacyloxy-2-alkyl-2-butene werden dadurch erhalten, daß man 1-Acyloxy-1.3-butadiene der Formel

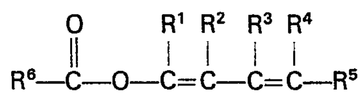

V,

in der die Reste $R^1$ bis $R^6$ die oben genannte Bedeutung haben, bei Temperaturen von 50 bis 180°C und Drucken von 1 bis 100 bar in Gegenwart von Palladium oder Platin enthaltenden Katalysatoren mit Sauerstoff und Carbonsäuren der Formel

$$R^7\text{—COOH} \qquad \text{VI},$$

in der $R^7$ die oben genannte Bedeutung hat, umsetzt.

Die Reaktion kann für den Fall der Herstellung von 1.1.4-Triacetoxy-2-methyl-2-buten durch folgende Formeln wiedergegeben werden (—OAc = —O—CO—$CH_3$):

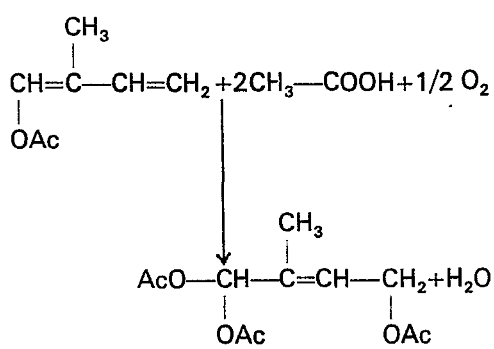

Das Ergebnis der erfindungsgemäßen Acyloxylierung von 1-Acyloxy-2-alkyl-1.3-butadienen und von 1.4-Diacyloxy-2-alkyl-1.3-butadienen, bei der die neuen 1.1.4-Triacyloxy-bzw. 1.1.4.4-Tetraacyloxy-2-alkyl-2-butene entstehen, ist überraschend, da nach dem in der DT—OS 2 200 124 beschriebenen Verfahren durch Umsetzung von 1-Acyloxy-1.3-butadienen mit Carbonsäuren und Sauerstoff in Gegenwart von Palladiumkatalysatoren 1.4-Diacyloxy-2-butene gebildet werden.

Die zur Herstellung der neuen 1.1.4-Triacyloxy-2-butene benötigten 1-Acyloxy-1.3-butadiene sind durch Acylierung von $\alpha,\beta$-ungesättigten Aldehyden mit Säureanhydriden, Säurehalogeniden, Ketenen oder Enolestern, wie 2-Acetoxypropen zugänglich (Houben-Weyl, Methoden der organischen Chemie, Band V/1 c, Seiten 184—192). So ist beispielsweise 1-Acetoxy-2-methyl-1.3-butadien aus Tiglinaldehyd ($\alpha$-Methyl-crotonaldehyd) oder $\alpha$-Äthyl-acrolein herstellbar.

Als Carbonsäuren der Formel VI werden vorzugsweise Ameisensäure oder solche mit Alkylresten aus 1 bis 3 Kohlenstoffatomen verwendet. Essigsäure ist aus wirtschaftlichen Gründen besonders bevorzugt. Die Carbonsäure wird im allgemeinen im Überschuß, bezogen auf das eingesetzte Dien, verwendet und dient damit gleichzeitig als Reaktionsmedium. Es ist aber auch möglich, die Umsetzung in einem unter den Reaktionsbedingungen inerten Lösungsmittel, wie Toluol, Xylol, Sulfolan oder Estern, wie Äthylacetat oder Butendiddiacetat durchzuführen.

Die Umsetzung der 1-Acyloxy-1.3-butadiene mit den Carbonsäuren zur Herstellung der 1.1.4-Tri- bzw. 1.1.4.4-Tetraacyloxy-2-alkyl-2-butene wird in Gegenwart von Saurestoff und den Katalysatoren bei Temperaturen zwischen 50 und 180°C durchgeführt. In der Gasphase liegt die Temperatur vorzugsweise bei 120 bis 150°C, in der Flüssigphase z.B. zwischen 50 und 110°C. Der Reaktionsdruck ist durch die Verfahrensweise gegeben und kann zwischen atmosphärischem Druck und 100 bar betragen. Man kann die Umsetzung chargenweise oder kontinuierlich durchführen, z.B. mit Fixbett, Wirbelbett oder Dreiphasenfließbett.

Als Palladium oder Platin enthaltende Katalysatoren kommen z.B. Trägerkatalysatoren in Betracht, die außer Palladium oder Platin mindestens eines der Elemente Antimon, Wismut, Tellur, Selen, Schwefel, Phosphor, Arsen, Eisen, Nickel, Kobalt und Kupfer enthalten. Das sind beispielsweise die in der DE—AS 2 217 452 genannten Trägerkatalysatoren, die außer Palladium noch mindestens eines, der Elemente Antimon, Wismut, Tellur und Selen enthalten, oder die in der DE—OS 2 417 658 beschriebenen Platin-Trägerkatalysatoren, die zusätzlich eines der Elemente Phosphor, Arsen, Antimon, Selen oder Tellur enthalten.

Die Verwendung von Trägerkatalysatoren ist besonders vorteilhaft. Man kann solche Trägerkatalysatoren auf an sich übliche Weise erhalten, beispielsweise dadurch, daß man einen Träger in einer Lösung dispergiert, die eine Palladium- oder Platinverbindung und eine oder mehrere Verbindungen der Übergangsmetalle enthält, das Lösungsmittel verdampft und den Rückstand in einem Gasstrom aus z.B. Wasserstoff oder Stickstoff, der mit einer reduzierenden Verbindung, wie Hydrazin, Methanol oder Formaldehyd beladen ist, reduziert. Die Reduktion des getrockneten Katalysators kann auch mit flüssigen Reduktionsmitteln geschehen. Man kann den Katalysator auch herstellen, indem man Träger und Lösung gemeinsam mit einem z.B. alkalischen Fällungsmittel behandelt und die Ausfällung isoliert und reduziert.

Eine sehr zweckmäßige Methode zur Herstellung der Katalysatoren besteht darin, daß man die Metalle aus den wäßrigen Lösungen ihrer Salze mit Reduktionsmittel, wie Formaldehyd, Hydrazin, u.a. bei einem geeigneten pH-Wert (vgl. z.B. J. Amer. Chem. Soc., Band 83, Seite 4916 (1961)) in Gegenwart der Träger ausfällt. Es empfiehlt sich die so erhaltenen rohen Katalysatoren noch in einem reduzierend wirkenden Gasstrom auf höhere Temperatur zu erhitzen.

Palladium bzw. Platin und die anderen Elemente können gleichzeitig oder in beliebiger Reihenfolge auf dem Träger abgeschieden werden: In manchen Fällen kann der Träger in Form einer löslichen Verbindung zugefügt und gemeinsam mit dem wirksamen Metall ausgefällt werden.

Man kann jedes Reduktionsverfahren anwenden, durch welches die verwendeten Elemente in den metallischen Zustand überführt werden. Als Trägermaterial kommen z.B. Aktivkohle, Bauxit, Bimsstein, Kieselgel, Kieselgur, Kieselsäure, Magnesia, Ton, Tonerde in Frage. Die Träger können gegebenenfalls durch eine übliche Vorbehandlung, z.B. mit einer Säure, für ihre Zweckbestimmung verbessert werden.

Die Wahl der zur Herstellung des Katalysators dienenden Palladium-bzw. Platinverbindung ist nicht entscheidend. So können z.B. halogenierte Palladium- bzw. Platinverbindungen, wie Palladiumchlorid, die Platinchloride, Salze einer organischen Säure wie Palladium- oder Platinacetat, die Nitrate, Oxide usw. verwendet werden. Man kann aber auch von anderen Palladium- oder Platinverbindungen, insbesondere komplexen Verbindungen, etwa Hexachloroplatinsäure, Natriumplatinsulfat, Ammoniumhexachlorpalatinat ausgehen. Auch die als weitere Katalysatorbestandteile zu verwendenden Übergangsmetalle können weitgehend in Form frei wählbarer Verbindungen zugegeben werden. Im allgemeinen wird man aus Zweckmäßigkeitsgründen zu löslichen Verbindungen greifen.

Gewöhnlich beträgt die Menge an den katalytisch wirksamen Metallen auf dem Träger 0,1 bis 20 Gew.%, bezogen auf das Katalysatorgewicht, jedoch sind auch größere und kleinere Konzentrationen möglich. Die Entscheidung, welche Menge die günstigste ist, kann auch von wirtschaftlichen Überlegungen bestimmt werden. Im Zweifel kann sie durch orientierende Versuche leicht gefunden werden.

Von den aus Palladium bzw. Platin einerseits und Eisen bzw. Kupfer andererseits bestehenden Trägerkatalysatoren, wie den Palladiumkatalysatoren der beschriebenen Art seien solche hervorgehoben, die Aktivkohle als Träger und — neben 1 bis 10% Palladium, bezogen auf das gesamte Katalysatorgewicht — etwa 1 bis 20% Kupfer und/oder 1 bis 10% Eisen enthalten. Höhere Konzentrationen an Palladium als die angegebenen können angewendet werden, bringen aber im allgemeinen keinen besonderen Vorteil.

Wenn die Wirksamkeit des Katalysators nach einer gewissen Betriebszeit abfällt, kann sie vielfach durch geeignete Verfahren wiederhergestellt werden. So kann beispielsweise eine Belegung des Katalysators mit polymeren Verbindungen mit Hilfe geeigneter Lösungsmittel oder durch vorsichtige Behandlung mit sauerstoffhaltigen Gasen rückgängig gemacht werden. Wenn die Aktivität des Katalysators durch Oxidationsvorgänge beeinträchtigt worden ist, kann eine Regenerierung vielfach

durch Behandlung mit reduzierend wirkenden Verbindungen wie Hydrazin, Formaldehyd, Wasserstoff, Kohlenoxid, Methanol(dampf) erreicht werden.

Sauerstoff wird in stöchiometrischer Menge oder im Überschuß verwendet. Es ist auch möglich, Sauerstoff im Gemisch mit inerten Gasen wie Stickstoff oder Kohlendioxid einzusetzen.

Die Umsetzung führt man bei diskontinuierlicher Arbeitsweise z.B. wie folgt durch: Einer Suspension des Katalysators in der jeweiligen Carbonsäure werden bei der Reaktionstemperatur und dem Reaktionsdruck Sauerstoff und das 1-Acyloxy-1.3-butadien zugeführt. Nach beendeter Zugabe wird gegebenenfalls noch nachgerührt. Durch das auf Raumtemperatur abgekühlte Reaktionsgemisch wird Stickstoff geleitet. Anschließend wird nach Abtrennung des Katalysators fraktioniert destilliert. Dabei werden nicht umgesetzte Ausgangsverbindungen und gegebenenfalls entstandene 4-Acyloxy-2-butenale von den Verfahrensprodukten abgetrennt. Unverbrauchtes 1-Acyloxy-1.3-butadien kann in die Acyloxylierungsstufe zurückgeführt werden.

Bei kontinuierlicher Arbeitsweise kann das Gemisch aus 1-Acyloxy-1.3-dien, Carbonsäure und Sauerstoff beispielsweise in Sumpf- oder Rieselfahrweise über den fest angeordneten Katalysator geleitet werden.

Nach dem erfindungsgemäßen Verfahren werden die neuen 1.1.4-Triacyloxy- und 1.1.4.4-Tetraacyloxyverbindungen mit hoher Selektivität erhalten.

Von den neuen Acyloxy-2-butenen sind die Verbindungen der Formel

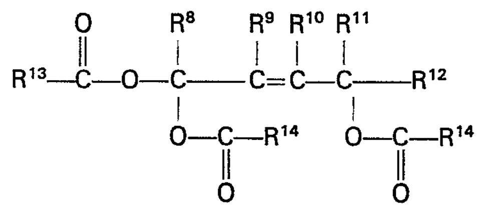

II,

in der $R^8$ und $R^{10}$ bis $R^{14}$ jeweils ein Wasserstoffatom oder einen Alkylrest mit 1 bis 3 C-Atomen und $R^9$ ein Alkylrest mit 1 bis 3 C-Atomen bedeuten, bevorzugt. Von ganz besonderem technischem Interesse sind die Verbindungen der Formel

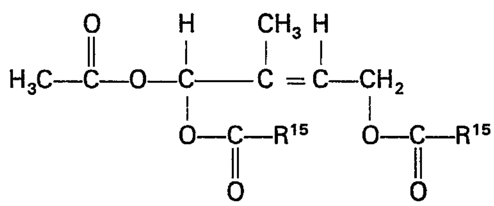

III,

in der $R^{15}$ ein Wasserstoffatom oder einen Methyl- oder Äthylrest bedeutet.

Da sich die neuen 1.1.4-Triacyloxy-2-butene leicht zu den entsprechenden 4-Acyloxy-2-butenalen hydrolysieren lassen, eröffnet diese Erfindung einen neuen vorteilhaften Syntheseweg, der z.B. vom Tiglinaldehyd (VII) über das durch bekannte Acetylierung erhältliche 1-Acetoxy-2-methyl-1.3-butadien (VIII) zu dem sehr gesuchten 4-Acetoxytiglinaldehyd (IX) führt.

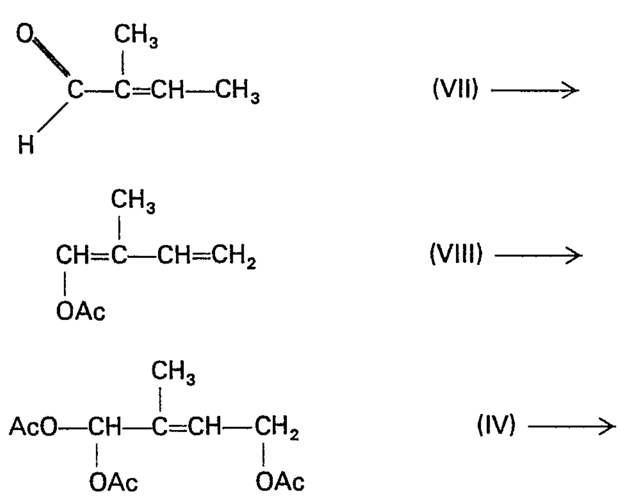

(VII)

(VIII)

(IV)

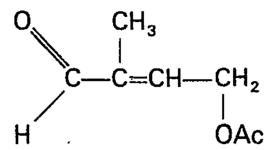 (IX)

Dieser neue Syntheseweg läuft auf eine Oxidation einer Methyl-, Methylen- oder Methingruppe in 2-Butenalen hinaus, da formal eine

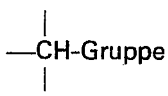

zu einer

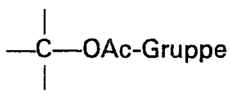

oxidiert wird.

Eine weitere vorteilhafte Ausführungsform der Erfindung betrifft somit die Verwendung der neuen 1.1.4-Triacyloxy-2-butene für die Herstellung von 4-Acyloxy-2-butenalen. Hierbei ist die Verwendung der Verbindungen der Formel III für die Herstellung der 4-Acyloxytiglinaldehyde der Formel IX, welche als $C_5$-Bausteine für die Synthese der Vitamin A-Acetat dienen, von besonderem technischem Interesse. Die Hydrolyse der 1.1.4-Triacyloxy-2-methyl-2-butene zum 4-Acyloxytiglinaldehyd wird auf an sich bekannte Weise, z.B. durch Behandlung mit Säuren oder Basen (Houben-Weyl, Methoden der Organischen Chemie, Band 7/1, Seite 444) vorgenommen. Man kann auch das Umsetzungsgemisch, das man bei der Herstellung der erfindungsgemäßen Acyloxy-2-butene der Formel III erhält, gegebenenfalls nach Abtrennung des Katalysators und der unverbrauchten Ausgangsverbindungen unmittelbar zu den entsprechenden 4-Acyloxy-2-butenalen hydrolysieren. Da bei der Acetoxylierung pro Mol gebildetes 1.1.4-Triacyloxy-2-buten ein Mol Wasser entsteht, ist eine für die Hydrolyse des Acylals ausreichende Menge Wasser vorhanden. Eine gewisse Menge an 4-Acyloxy-2-butenal ist daher auch schon nach der Acyloxylierung neben 1.1.4-Triacyloxy-2-buten gaschromatographisch nachweisbar.. Zur Beschleunigung der Hydrolyse kann man gegebenenfalls Wasser und/oder Verseifungskatalysatoren zusetzen und bei Normaldruck zwischen 20°C und der Siedetemperatur der jeweiligen Carbonsäure oder über diesem Siedepunkt unter Druck arbeiten.

Die in den Beispielen genannten Teile sind Gewichtsteile.

### Beispiel 1

*1.1.4-Triacetoxy-2-methyl-2-buten (Pd-Cu-Katalysator)*

a) Herstellung des Katalysators:

4,48 Teile Kupferpulver, gelöst in 42 Volumenteilen 33%iger Salpetersäure, werden bei Raumtemperatur zu 5,31 Teilen Palladiumacetat, gelöst in 75 Volumenteilen 50%igem Äthanol hinzugefügt. Diese Salzlösung wird bei Raumtemperatur zu 50 Teilen Aktivkohle (0,3 bis 0,5 mm, 35 bis 50 mesh) zugegeben, die vorher bei Raumtemperatur mit 160 Volumenteilen 15%iger Salpetersäure versetzt, auf 70°C erwärmt, 5 Stunden bei dieser Temperatur gerührt, nach dem Abkühlen auf einer Glasfilternutsche gesammelt, bis zur Erreichung eines pH-Wertes von 7—8 mit Wasser gewaschen und 16 Stunden im Vakuumtrockenschrank getrocknet worden war. Das Gemisch aus Salzlösung und Aktivkohle wird bei 85°C am Rotationsverdampfer (Wassertrahlvakuum) bis zur Trockene eingeengt.

Der Kontakt wird 2 Stunden bei 150°C im Vakuumtrockenschrank, dann 2 Stunden bei 150°C im Röhrenofen unter strömendem Stickstoff getrocknet. Anschließend wird der Kontakt 6 Stunden bei 200°C, dann 6 Stunden bei 400°C mit bei Raumtemperatur mit Methanol gesättigtem Stickstoff und schließlich 0,5 Stunden mit Wasserstoff (20 000 Volumenteile/Stunde) bei 800°C aktiviert. Man läßt unter strömendem Stickstoff auf Raumtemperatur abkühlen und bewahrt den Kontakt in einer gut verschlossenen Flasche unter Argon auf.

Die Röntgenanalyse des Katalysators zeigt, daß eine $PdCu_3$ Phase mit wenig PdCu vorliegt.

b) Herstellung von 1.1.4-Triacetoxy-2-methyl-2-buten

In einem Dreihalskolben mit Begasungsrührer, Innenthermometer, Rückflußkühler, Gaseinleitungsrohr und Tropftrichter werden 15 Teile des Katalysators, suspendiert in 600 Teilen Eisessig, vorgelegt und unter strömendem Stickstoff auf 95°C (Ölbad) erwärmt. Dann werden bei dieser Temperatur, gleichmäßig verteilt über 4 Stunden, gleichzeitig 67,5 Teile 1-Acetoxy-2-methyl-1.3-butadien aus dem Tropftrichter und 12 000 Volumenteile Sauerstoff durch das Gaseinleitungsrohr zugeführt. Man rührt nach beendeter Zugabe der Ausgangsprodukte 15 Minuten unter Durchleiten von Stickstoff nach, läßt das Reaktionsgemisch auf Raumtemperatur abkühlen und saugt den Katalysator

auf einer Glasfilternutsche ab. Es werden so 668 Teile Reaktionsprodukt erhalten, das nach gaschromatographischer Analyse folgende Zusammensetzung besitzt:

| | |
|---|---|
| (E)- + (Z)-1.1.4-Triacetoxy-2-methyl-2-buten | : 6,50 Gew.% |
| (E)- + (Z)-4-Acetoxy-tiglinaldehyd | : 1,90 Gew.% |
| unumgesetztes 1-Acetoxy-2-methyl-1.3-butadien | : 3,96 Gew.% |
| Tiglinaldehyd | : 0,26 Gew.% |
| Diacetoxy-2-methyl-1.3-butadien | : 0,30 Gew.% |
| unbekannte Verbindungen | : 0,50 Gew.% |
| Essigsäure | : Rest |

Durch fraktionierte Destillation des Reaktionsgemisches erhält man 39,5 Teile 1.1.4-Triacetoxy-2-methyl-2-buten vom Siedepunkt 104—111°C/0,3 mbar ($n_D^{20}$ = 1,4493) (30,2% Umwandlung). Die Ausbeute an 1.1.4-Triacetoxy-2-methyl-2-buten und 4-Acetoxytiglinaldehyd beträgt — unter Berücksichtigung von gaschromatographisch bestimmtem Tiglinaldehyd und 1-Acetoxy-2-methyl-1.3-butadien als Ausgangsprodukten — zusammengenommen 87,7%.

Beispiel 2

*1.1.4-Triacetoxy-2-methyl-2-buten (Pd-Te-Katalysator)*

Wie in Beispiel 1 beschrieben, werden 15 Teile eines nach der DT—AS 2 217 452 hergestellten Palladium-Tellur-Katalysators mit Aktivkohole (0,2—0,4 mm) als Trägermaterial (5% Pd, 1% Te), suspendiert in 600 Teilen Eisessig, bei 95°C innerhalb von 2 Stunden mit 34 Teilen 1-Acetoxy-2-methyl-1.3-butadien und 6 000 Volumenteilen Sauerstoff umgesetzt. Nach der in Beispiel 1b angegebenen Aufarbeitung und Destillation werden neben nicht umgesetztem Ausgangsmaterial 18,6 Teile 1.1.4-Triacetoxy-2-methyl-2-buten vom Siedepunkt 105 bis 112°C/0,4 mbar ($n_D^{20}$ = 1,4492) (28,2% Umwandlung) erhalten.

Beispiel 3

*1.1.4-Triacetoxy-2-methyl-2-buten (Pt-Te-Katalysator)*

Man verfährt wie in Beispiel 2 beschrieben, verwendet jedoch 15 Teile eines nach der DT—OS 2 417 658 hergestellten Platin-Tellur-Katalysators mit Aktivkohle (0,2—0,4 mm) als Trägermaterial (4,9% Pt, 1,0% Te). Es werden 8,7 Teile 1.1.4-Triacetoxy-2-methyl-2-buten vom Siedepunkt 114 bis 120°C/0,7 mbar ($n_D^{20}$ = 1,4489) (13,2% Umwandlung) erhalten.

Beispiel 4

*1.1.4-Triacetoxy-2-methyl-2-buten (Pd-Cu-Katalysator)*

In eine Lösung von 63 Teilen 1-Acetoxy-2-methyl-1.3-butadien in 600 Teilen Eisessig, in der 25 Teile des nach Beispiel 1a hergestellten Katalysators suspendiert sind, werden bei 95°C innerhalb von 4 Stunden 12 000 Volumenteile Sauerstoff eingeleitet. Man arbeitet wie in Beispiel 1b beschrieben auf und erhält durch fraktionierte Destillation 71,8 Teile 1.1.4-Triacetoxy-2-methyl-2-buten (58,8% Umwandlung) vom Siedepunkt 118 bis 124°C/0,7 mbar und 19,5 Teile 4-Acetoxytiglinaldehyd (27,4% Umwandlung) vom Siedepunkt 102 bis 107°C/25 bis 27 mbar.

Beispiel 5

*Verwendung von 1.1.4-Triacetoxy-2-methyl-2-buten für die Herstellung von 4-Acetoxy-tiglinaldehyd*

In gleicher Weise wie in Beispiel 1b setzt man 67,5 Teile 1-Acetoxy-2-methyl-1.3-butadien in 600 Teilen Eisessig mit 12 000 Volumenteilen Sauerstoff in Gegenwart von 15 Teilen des nach Beispiel 1a hergestellten Katalysators um. Nach beendeter Zugabe des Diens werden innerhalb von 2 Stunden weitere 6 000 Volumenteile Sauerstoff bei 95°C eingeleitet. Dann rührt man unter Durchleiten von Stickstoff 15 Minuten nach, läßt das Reaktionsgemisch auf Raumptemperatur abkühen, saugt den Katalysator ab, versetzt das Filtrat mit 28,9 Teilen Wasser und erhitzt 10 Stunden lang zum Rückfluß. Durch fraktionierte Destillation erhält man 49,4 Teile (E)-4-Acetoxy-tiglinaldehyd (65% Umwandlung) vom Siedepunkt 105—107°C/27 mbar, $n_D^{20}$ = 1,4640.

**Patentansprüche**

1. Acyloxy-2-butene der Formel

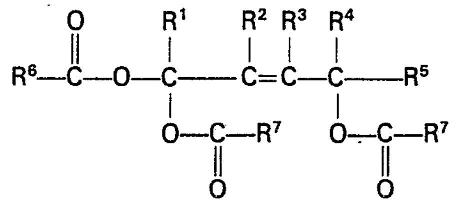

I,

in der $R^1$, $R^3$ und $R^4$ jeweils ein Wasserstoffatom oder einen Alkylrest mit 1 bis 5 C-Atomen, $R^2$ einen Alkylrest mit 1 bis 5 C-Atomen, $R^5$ ein Wasserstoffatom, einen Alkylrest mit 1 bis 5 C-Atomen oder eine $R^6$—CO—O-Gruppe und $R^6$ und $R^7$ jeweils ein Wasserstoffatom, einen Alkylrest mit 1 bis 5 C-Atomen oder einen Phenyl- oder Cyclohexylrest bedeuten.

2. Acyloxy-2-butene der Formel

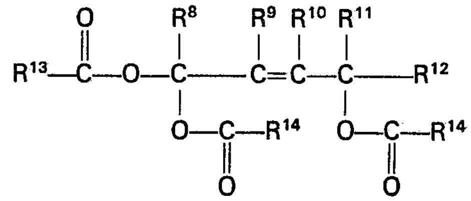

II,

in der $R^8$ und $R^{10}$ bis $R^{14}$ jeweils ein Wasserstoffatom oder einen Alkylrest mit 1 bis 3 C-Atomen und $R^9$ einen Alkylrest mit 1 bis 3 C-Atomen bedeuten.

3. 1.1.4-Triacyloxy-2-methyl-2-butene der Formel

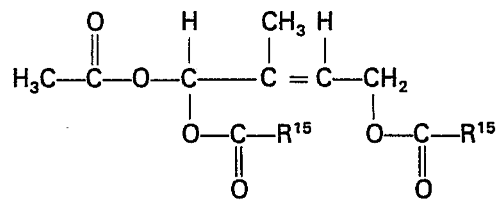

III,

in der $R^{15}$ ein Wasserstoffatom oder einen Methyl- oder Äthylrest bedeutet.

4. 1.1.4-Triacetoxy-2-methyl-2-buten der Formel

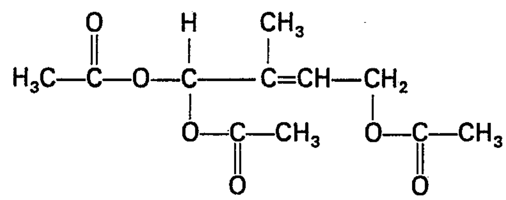

IV,

5. Verfahren zur Herstellung von Acyloxy-2-butenen nach Anspruch 1, *dadurch gekennzeichnet,* daß man 1-Acyloxy-1,3-butadiene der Formel

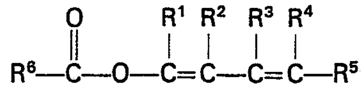

V,

in der die Reste $R^1$ bis $R^6$ die in Anspruch 1 genannte Bedeutung haben, bei Temperaturen von 50 bis 180°C und Drucken von 1 bis 100 bar in Gegenwart von Palladium oder Platin enthaltenden Katalyatoren mit Sauerstoff und Carbonsäuren der Formel

$$R^7\text{—COOH} \qquad \text{VI,}$$

in der $R^7$ die in Anspruch 1 genannte Bedeutung hat, umsetzt.

6. Verwendung der 1.1.4-Triacyloxy-2-methyl-2-butene nach Anspruch 3 für die Herstellung von 4-Acyloxytiglinaldehyd.

**Revendications**

1. Acyloxy-2-butène de formule

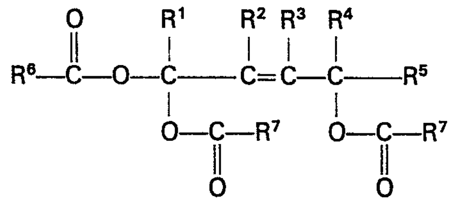

I,

dans laquelle $R^1$, $R^3$ et $R^4$ représentent chacun un atome d'hydrogène ou un reste alkyle en C1—C5, $R^2$ représente un reste alkyle en C1—C5, $R^5$ représente un atome d'hydrogène, un reste alkyle en C1—C5 ou un groupe $R^6$—CO—O— et $R^6$ et $R^7$ représentent chacun un atome d'hydrogène, un reste alkyle en C1—C5, un reste phényle ou cyclohexyle.

2. Acyloxy-2-butènes répondant à la formule

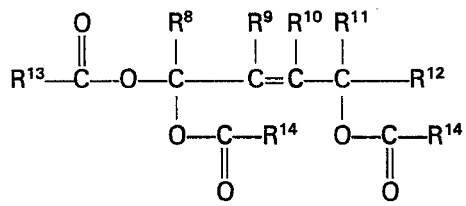

II,

dans laquelle $R^8$ et $R^{10}$ à $R^{14}$ représentent chacun un atome d'hydrogène ou un reste alkyle en C1—C3 et $R^9$ représente un reste alkyle en C1—C3.

3. 1,1,4-triacyloxy-2-méthyl-2-butènes de formule

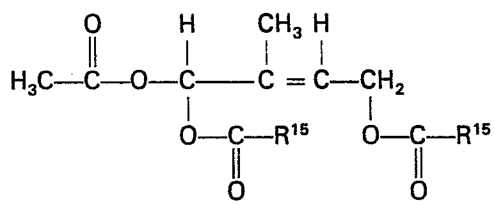

III,

dans laquelle $R^{15}$ représente un atome d'hydrogène ou un reste méthyle ou éthyle.

4. 1,1,4-triacétoxy-2-méthyl-2-butène de formule

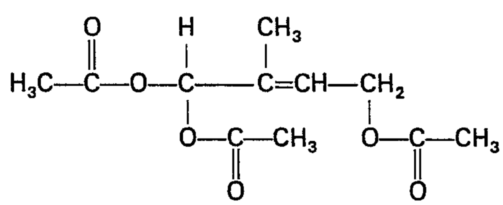

IV,

5. Procédé de préparation des acyloxy-2-butènes selon la revendication 1, caractérisé en ce que l'on fait réagir des 1-acyloxy-1,3-butadiènes répondant à la formule

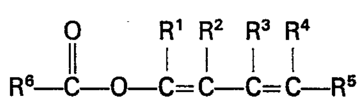

V,

dans laquelle les symboles $R^1$ à $R^6$ ont les significations indiquées dans la revendication 1, à des températures de 50 à 180°C et des pressions de 1 à 100 bars, en présence de catalyseurs contenant du palladium ou du platine, avec de l'oxygène et des acides carboxyliques répondant à la formule

$$R^7\text{—COOH} \qquad \text{VI,}$$

dans laquelle $R^7$ a la signification indiquée dans la revendication 1.

6. Utilisation des 1,1,4-triacyloxy-2-méthyl-2-butènes selon la revendication 3 dans la préparation des aldéhydes 4-acyloxy-tigliques.

**Claims**

1. Acyloxy-2-butenes of the formula

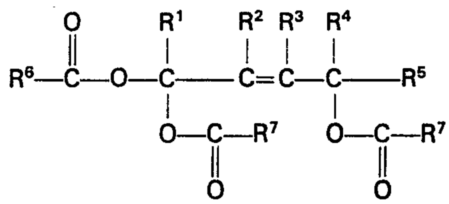

I

where $R^1$, $R^3$ and $R^4$ are each hydrogen or alkyl of 1 to 5 carbon atoms, $R^2$ is alkyl of 1 to 5 carbon atoms, $R^5$ is hydrogen, alkyl of 1 to 5 carbon atoms or $R^6$—CO—O, and $R^6$ and $R^7$ are each hydrogen, alkyl of 1 to 5 carbon atoms, phenyl or cyclohexyl.

2. Acyloxy-2-butenes of the formula

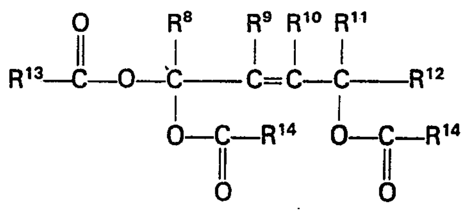

II

where $R^8$ and $R^{10}$ to $R^{14}$ are each hydrogen or alkyl of 1 to 3 carbon atoms and $R^9$ is alkyl of 1 to 3 carbon atoms.

3. 1,1,4-Triacyloxy-2-methyl-2-butenes of the formula

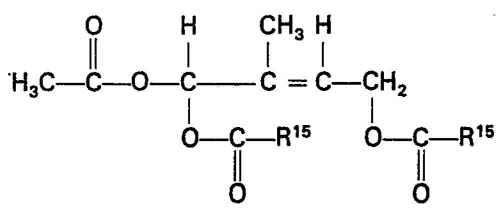

III

where $R^{15}$ is hydrogen, methyl or ethyl.

4. 1,1,4-Triacetoxy-2-methyl-2-butene of the formula

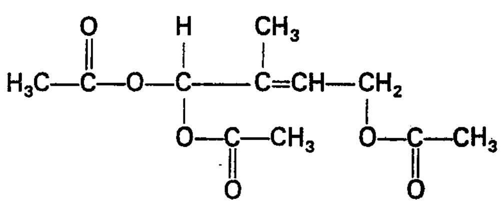

IV

5. Process for the preparation of an acyloxy-2-butene as claimed in claim 1, wherein a 1-acyloxy-1,3-butadiene of the formula

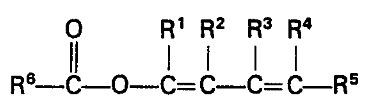

V

where the radicals $R^1$ to $R^6$ are as defined in claim 1, is reacted with oxygen and a carboxylic acid of the formula

$$R^7\text{—COOH} \qquad \text{VI}$$

where $R^7$ is as defined in claim 1, at from 50 to 180°C and a pressure of from 1 to 100 bar in the presence of a catalyst containing palladium or platinum.

6. Use of the 1,1,4-triacyloxy-2-methyl-2-butenes as claimed in claim 3 for the preparation of 4-acyloxytiglic aldehyde.